# EUROPEAN PATENT APPLICATION

(11) **EP 4 151 618 A1**
(43) Date of publication of application: **22.03.2023**
(21) Application number: 21197669.1
(22) Date of filing: 20.09.2021
(51) Int. Cl.: C07C 209/84, C07C 211/09, C07C 263/10, C07C 265/14

(54) **OBTAINING ALIPHATIC AMINES FROM COMPOSITIONS**

(71) Applicant: Covestro Deutschland AG, 51373 Leverkusen (DE); Genomatica, Inc., San Diego CA 92121 (US)
(72) Inventor: MERKEL, Michael, 40223 Düsseldorf (DE); LEIMBRINK, Mathias, 44139 Dortmund (DE); STEFFENS, Friedhelm, 51373 Leverkusen (DE)
(74) Representative: Scholz, Volker

(57) **Abstract**

The invention relates to a method for obtaining an amine from a composition by means of distillation and a composition obtainable by that method. The invention also relates to a method for the production of an isocyanate from the composition obtainable.

## Description

The invention relates to a method for obtaining an amine from a composition by means of distillation and a composition obtainable by that method. The invention also relates to a method for the production of an isocyanate from the composition obtainable.

Amines are important basic materials in the chemical industry. They are formed for example when ammonia is reacted with alcohols or aldehydes in the presence of hydrogen, where as a further reaction product water *inter alia* forms, and this frequently combines with the amine produced to form an amine/water mixture with an azeotropic boiling point. Methods for separating mixtures of this kind are described in EP1312599A1 and EP1312600A1 for example. The disclosure there is of the separation of low boilers from the mixture by distillation, the separation of high boilers from the mixture by distillation, the extraction of the amine-containing mixture and the distillation of the aqueous organic phase obtained in this way so that anhydrous amine and an amine/water azeotrope are obtained.

Aliphatic diamines in particular are an important starting material for the production of polymers. They are used for example for the production of polyamides by polycondensing the diamine with dicarboxylic acids. Polyamide-6.6 (PA66), for example, is generated from hexamethylenediamine and adipic acid. Another important use is the phosgenation of the diamines to diisocyanates, which can then be converted in a further step into polyurethanes, polyureas or polyisocyanurates for example. Phosgenation places special demands on the purity of the aliphatic diamine.

Certain requirements to be met by the diamine are known, for example, for the production of hexamethylene diisocyanate from hexamethylenediamine. CN103922969B discloses that secondary components with lower boiling points, such as tetrahydroazepine, 1-amino-2-cyano-1-cyclopentene or 6-aminocapronitrile, should only be present in very small amounts. EP2060560B1 describes that isocyanates with particularly bright colours can be produced from amines whose PRI value, i.e. the content of polarographically reducible compounds, is less than 60 mol-ppm.

One example of the production of polyamides is by heating aqueous solutions of diamines and dicarboxylic acids. In this case, the solution is heated under pressure, with the pressure being kept constant by deliberately releasing surplus water vapour. In order to complete the polycondensation, the water must be removed from the reaction mixture completely. Effluent containing diamine is formed in the process, which has to be treated in a complex manner. CN101993178(A) describes such an effluent treatment method for effluents containing hexamethylenediamine, which involves the anaerobic and aerobic treatment of the effluent. One disadvantage of the method is that the hexamethylenediamine contained is destroyed in the process and thus cannot be further recycled.

Other aliphatic diamines are obtained for example by the hydrogenation or reductive amination (also known as aminating hydrogenation) of appropriate oxygen-containing compounds. As described in WO2012076315A1, this can for example be the reductive amination of 3-cyano-3,5,5-trimethylcyclohexanone to 3-aminomethyl-3,5,5- trimethylcyclohexylamine (IPDA) or also the hydrogenation of compounds containing nitrogroups. In the process, not only the desired diamine but also water known as water of reaction is formed. This can then be separated from the amine in various ways. If there is a miscibility gap or if one can be caused by adding further substances (e.g. salts or solvents), an obvious step is first to carry out phase drying and later to dry the organic amine phase further by distillation. If there is no miscibility gap, it is customary to remove by distillation the entire water needing to be removed. In both cases, a stream of effluent arises, in which some of the diamine is still dissolved.

WO2019166482A1 describes the distillation treatment of hexamethylenediamine using a dividing wall column, in which a stream enriched in low boilers is recovered at the head, a purified hexamethylenediamine stream is recovered as a side stream, and a stream enriched in high boilers is recovered at the bottom of the column. Compared to distillation in 2 columns, an energy saving of up to 20% is mentioned. This, however, relates to a hexamethylenediamine from which water has already been removed and which has only a small content of impurities, so that a large proportion of the energy required has already been consumed beforehand and is not taken into account in this figure. There is no discussion of further ways of saving energy, such as by using the condensation heat of the stream of steam. A further source of solutions of diamines in water or organic solvents is the recycling of plastics, such as polyurethanes or polyamides, which is playing an ever more important role in industry. If those plastics are hydrolysed chemically, aqueous solutions of diamines frequently result, from which the diamines must be recovered.

Classic purification sequences for obtaining diamines from an aqueous solution generally involve removing water as one of the first steps in the treatment process. Distillation in a multiple column is known to be a suitable method for removing water, so that water is separated over the head and an amine from which most of the water has been removed is left at the bottom of the column.

Pentamethylenediamine is produced industrially by biotechnological means and is formed as a mixture with water in the production process. EP2684867A1, for example, describes a method for the production of pentamethylenediamine in which the diamine is extracted in a liquid phase extraction process from the aqueous phase that has optionally been concentrated by distillation beforehand. Non-halogen aliphatic solvents, preferably straight chain alcohols with 4 to 7 carbon atoms, are recommended as extractants. These extractants must subsequently be separated from the product by distillation so that they can be used in the extraction process again. The fact that the heat of evaporation of the extractants is usually lower than that of water makes a certain energy saving possible in principle. This is counterbalanced, however, by the need for an additional process step and the introduction of large quantities of a further substance into the process. For the distillation processes used to concentrate the aqueous raw product or to isolate the pentamethylenediamine from the extract, the use of a multiple column at a pressure of between 0.1 kPa and normal pressure is described in each case. No further steps to enhance the energy efficiency in these distillation processes are mentioned.

EP3235804A1 also describes the extraction of pentamethylenediamine from an aqueous phase. Following extraction, however, a reduction process is also carried out here in order to eliminate 2,3,4,5-tetrahydropyridines, which are blamed for later discolorations of the polymers produced starting from the PDA. For this reason, a requirement placed on the extractants is that they should remain inert in the chemical hydrogenation process. Once again, alcohols such as ethanol or butanol are preferred as extractants. No details are disclosed concerning the distillation process and possible ways of carrying it out efficiently.

One possible way of saving energy in distillation processes is the thermal integration of two columns. One such approach was described by O. Annakou and P. Mizsey (Ind. Eng. Chem. Res. 1996, 35, 1877-1885, Rigorous Comparative Study of Energy-Integrated Distillation). While that article mentions the fundamental possibility of saving energy, it nevertheless only discusses mixtures of alkanes (butane to heptane), all of which have a boiling point lower than 100° C and, within the mixtures considered, also exhibit only a moderate spread of boiling points of no more than 62 K (pentane/hexane/heptane). One consequence of this is that the distillation can be performed under very mild conditions. Furthermore, the alkanes mentioned are in any case very stable under thermal loads. There was no demonstration that the principle could be applied to thermally less stable compounds such as amines. If there is an excess of the component with a lower boiling point in the mixture to be separated, or if it is a compound with a high evaporation enthalpy, such as water, the saving that can be achieved in the set-ups described is limited by the fact that the two columns have extremely different energy requirements, as a result of which either too much (forward match) or too little (reverse match) energy is available from the steam condensation to heat the other column.

Finally, a method is described in EP1602640B1 in which water from aqueous amine solutions, such as those produced in the hydrogenation of nitroaromatics, is separated in two distillation columns arranged in series. In the process, one of the columns is operated at 2 to 20 bar and the other at 0.1 to 10 bar. The condensation heat of the steam emerging from the column operated at higher pressure is used to evaporate water from the bottom of the column operated at lower pressure. The problem described above regarding the lower thermal stability of the amines is avoided in two ways. On the one hand, the method is limited to aromatic amines, which are generally known to have greater stability than aliphatic amines (see also EP1754698A2, paragraph [0004]), and on the other hand it dispenses with evaporating the aromatic amine completely in order to keep the thermal load small. The amine therefore substantially occurs as a non-evaporated bottom product, and even water is only removed incompletely from the starting solution. Impurities with higher boiling points remain in the amine in this method.

Starting from this state of the art, the problem underlying the present invention was to provide an improved, economically and ecologically meaningful method with which amines can be isolated from a composition in a quality suitable for the production of isocyanate by phosgenating those amines.

This problem has been solved by a method for producing an amine comprising the steps of:
a) providing a composition comprising the amine, at least one compound having a lower boiling point than the amine and at least one other compound having a higher boiling point than the amine,
b) distilling the composition in a thermally integrated distillation apparatus comprising a first distillation column, an evaporator-condenser with an evaporation space and a condensing space and a second distillation column, wherein
   - the composition of step a) is introduced as feed into the first distillation column operating at a head pressure in the range from 1 to 23 bar(a),
   - a bottoms liquid depleted in the lower boiling compound from the first distillation column is introduced into the evaporation space of the evaporator-condenser, is partially evaporated and is then separated into a gaseous feed, which is introduced into the second distillation column, and a residual stream,
   - the second distillation column is operating at a head pressure in the range from 0.05 to 3.8 bar(a) and at least 0.5 bar which is at least 0.5 bar lower than the head pressure in the first distillation column,
   - a first stream of the steam from the first distillation column is introduced into the condensing space of the evaporator-condenser, is at least partially condensed there so that a condensate forms and the condensation heat is used to evaporate the bottoms liquid of the first column in the evaporation space of the evaporator-condenser partially, and
   - a product stream including the amine is removed as a side stream below the feed or as a bottoms product from the second column.

In accordance with the invention, the expressions "comprising", "containing" or "including" preferably mean "consisting substantially of" and particularly preferably "consisting of".

Expressions such as *"an evaporation space", "a condensing space", "a first partial stream and* a *second partial stream"* or "a *first distillation column and* a *second distillation column"* or "a *first part and* a *second part"* are always to be understood, unless specifically stated otherwise, as open wording, which does not rule out the presence of further (third, fourth,...) partial streams, distillation columns or parts.

*Statements concerning the content of organic compounds* in the context of the present invention refer, unless stated otherwise, to values determined by gas chromatography. The skilled person is familiar with the quantitative evaluation of gas chromatograms. The skilled person is likewise familiar with any methods that might perhaps be needed to determine the *water content.* The methods known in the art can also be used in the context of the present invention. In case of doubt, the water content determined by Karl Fischer titration is decisive. For Karl Fischer titration in general, see Jander, Jahr, Massanalyse, 17th ed., de Gruyter, Berlin (2009), p. 279 to p. 282).

Numerical values provided herein without decimal places refer in each case to the full value stated with one decimal place. "23 bar(a)", for example, stands for "23.0 bar(a)".

Numerical ranges shown in the format "in/from x to y" include the values given. If a number of preferred numerical ranges are shown in this format, it goes without saying that all ranges arising by combining the various end points are likewise encompassed.

*"Distillation"* in the present case is understood to mean a thermal separation process used to recover evaporable substances, preferably liquids, from a composition. The separated vapours are subsequently precipitated, usually by condensation. The term encompasses in particular repeated evaporation and condensation using a column (distillation column) with a plurality of separating stages. Processes of this kind in which several distillation steps are arranged in series in a column should strictly speaking be called rectification, but it has become standard practice likewise to refer to them as distillation for the purpose of simplification, and this practice will also be adopted herein. The advantage of these processes is the powerful separating effect and the possibility of operating the plant continuously.

The expression *"back feed stream"* or *"back feed"* for short refers to that part of the condensed head product of a distillation column which is not removed as a product stream, but is returned to the distillation column or optionally to a different distillation column.

In a second embodiment of the invention, the bottoms liquid from the first distillation column depleted in the lower boiling compound is introduced into the second distillation column. The evaporator-condenser is then used not for evaporating the feed to the second distillation column, but rather as a circulation evaporator for heating the second distillation column. The subject-matter of the invention is therefore also a method for obtaining an amine comprising the steps of:
a) providing a composition comprising the amine, at least one compound having a lower boiling point than the amine and at least one other compound having a higher boiling point than the amine,
b) distilling the composition in a thermally integrated distillation apparatus comprising a first distillation column, an evaporator-condenser with an evaporation space and a condensing space and a second distillation column, wherein
   - the composition of step a) is introduced as feed into the first distillation column operating at a head pressure in the range from 1 to 23 bar(a),
   - the bottoms liquid from the first distillation column depleted in the lower boiling compound is introduced as feed into the second distillation column which is operating at a head pressure in the range from 0.05 to 1.5 bar(a) which is at least 0.5 bar below the head pressure in the first distillation column,
   - a liquid stream is removed from the bottoms of the second distillation column and is introduced into the evaporation space of the evaporator-condenser, is partially evaporated therein and is fed back into the column,
   - a liquid residual stream which may optionally comprise solids is removed and discharged from the evaporation space of the evaporator-condenser, from the bottom of the column or as part of the bottoms liquid removed there,
   - a steam stream from the first distillation column is introduced into the condensing space of the evaporator-condenser, and is at least partially condensed therein, thus forming a condensate, and the condensation heat is used to partially evaporate the liquid stream removed from the bottom of the column, and
   - a product stream including the amine is removed either as a partial stream from the steam produced in the evaporation space of the evaporator-condenser or is removed below the feed as a side stream from the second distillation column.

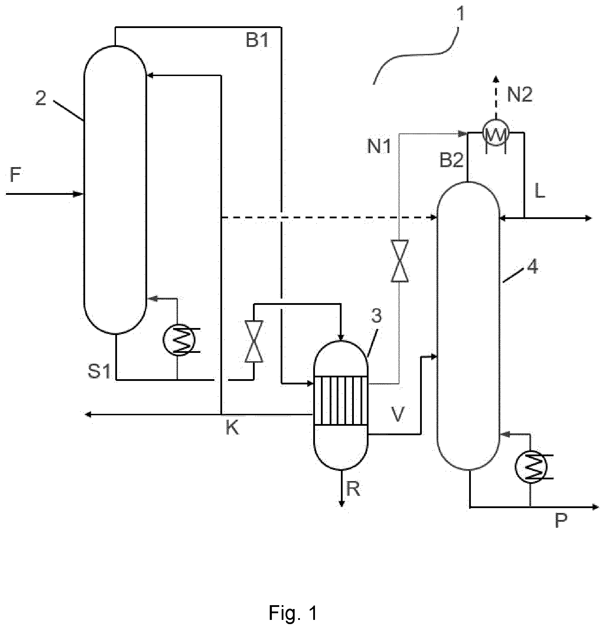

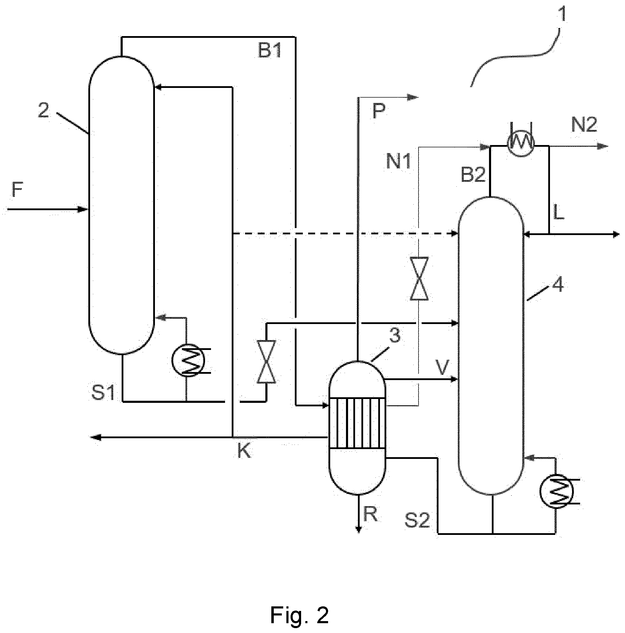

In the following description of the invention, reference will be made to the drawings of Fig. 1 and Fig. 2 for illustration purposes, though without limiting the invention thereto.

Fig. 1 schematically illustrates a distillation apparatus which is suitable for performing the method of the invention according to the first embodiment. Fig. 2 schematically illustrates a distillation apparatus which is suitable for performing the method of the invention according to the second embodiment.

The individual process steps will be explained in more detail below.

### Step a)

As already described in the introduction, there are various processes in industrial chemistry in which compositions including amines are produced and which are in principle suitable for obtaining the amine according to the present method. Thus the composition to be provided in step a) can basically originate from any process. It may for example be a diaminotoluene-water mixture from the hydrogenation of dinitrotoluene, an HDA-water mixture from a process of recycling PA66, especially by acid hydrolysis, an IPDA-containing effluent from an IPDA production process or a solution of PDA from the fermentation production of PDA. The latter may, for example, be a raw solution in water, from which cellular components have already been separated. It may equally well be a solution of an amine in a solvent with a lower boiling point than the amine, for example in an alcohol such as butanol or hexanol, with the aid of which the amine has already been extracted from an aqueous phase, such as is produced for example in the fermentation production of PDA or in the chemolysis, especially the basic hydrolysis, of PA66 or other polyamides.

Suitable compositions contain not only the amine to be obtained, but at least also one compound having a lower boiling point than the amine, which may be either a compound which itself has a lower boiling point than the amine or one which together with the amine forms an azeotrope with a lower boiling point. It may for example be water or organic solvents such as monohydric alcohols, e.g. butanol, pentanol, hexanol or heptanol, esters or chlorinated hydrocarbons such as chloroform or dichloroethane. Of course mixtures of different substances with a lower boiling point than the amine may also occur. In the present case, the compound with a lower boiling point which has the greatest share by weight in the totality of the substances with lower boiling points than the amine in the composition is referred to as *"the compound with a lower boiling point".* It preferably accounts for at least 60 wt.-%, preferably at least 80 wt.-% and particularly preferably at least 90 wt.-% of the totality of the substances in the composition with lower boiling points than the amine. In addition, further secondary components with lower boiling points will also occur as a rule, which are different from the compound with a lower boiling point and whose content is considerably lower than that the of the compound with a lower boiling point. Some of these secondary components with lower boiling points usually account for no more than 5 wt.-% of the total amount of compounds having a lower boiling point than the amine. These may for example be ammonia, alkyl or alkenyl amines, alcohols, ethylene glycol, cyclic amines such as pyrrolidine, piperidine, azepane, imines, cylic imines such as tetrahydropyridine or tetrahydroazepine, or residues of solvents such as hydrocarbons or halogenated hydrocarbons or, if it is a solution of the amine in an organic solvent, also water. In addition, gases such as nitrogen or carbon dioxide may be present in the composition in dissolved or bound form.

In a preferred embodiment of the method of the invention, the composition provided in method step a) comprises ≥ 40 wt.-% and ≤ 99 wt.-%, preferably ≥ 60 wt.-% and ≤ 98 wt.-% and particularly preferably ≥ 70 wt.-% and ≤ 95 wt.-% of the at least one compound having a lower boiling point than the amine. Furthermore, the composition preferably includes ≥ 1 wt.-% and ≤ 50 wt.-%, particularly preferably ≥ 2% wt.-% and ≤ 40 wt.-% and most particularly preferably ≥ 4 wt.-% and ≤ 25 wt.-% of the aliphatic amine. The composition preferably includes ≥ 0.01 wt.-% and ≤ 10 wt.-%, particularly preferably ≥ 0.1 wt.-% and ≤ 5 wt.-% and most particularly preferably ≥ 0.3 wt.-% and ≤ 3 wt.-% of the at least one compound having a higher boiling point than the amine. The proportion of further secondary components with lower boiling points is preferably in the range between ≥ 0.01 wt.-% and ≤ 10 wt.-%, particularly preferably between ≥ 0.1 wt.-% and ≤ 5 wt.-%, most particularly preferably between ≥ 0.3 wt.-% and ≤ 3 wt.-%.

It is especially advantageous if the at least one compound having a lower boiling point than the amine is water *(the compound with a lower boiling point).* Water has a particularly high evaporation enthalpy. The high energy requirements resulting from this for separating the water by distillation conversely enables a particularly high saving by using of the method of the invention. In this embodiment, the composition thus preferably comprises ≥ 40 wt.-% and ≤ 99 wt.-%, more preferably ≥ 60 wt.-% and ≤ 98 wt.-% and particularly preferably ≥ 70 wt.-% and ≤ 95 wt.-% water.

As amines, all the aromatic, aliphatic, cycloaliphatic or araliphatic compounds with primary amino groups, i.e. NH₂ groups, with which the skilled person is familiar, can be used. According to the invention however, those compounds are preferred which have at least 2, particularly preferably 2 or 3 NH₂ groups. These are particularly preferably aliphatic, cycloaliphatic or araliphatic amines. According to the invention, those amines are most particularly preferred which have exactly 2 aliphatically, cycloaliphatically and/or araliphatically bound NH₂ groups.

Suitable aromatic, aliphatic, cycloaliphatic or araliphatic amines are for example selected from the group consisting of diaminotoluene, n-butylamine, iso-butylamine, n-hexylamine, butane-1,4-diamine, pentane-1,5-diamine (PDA), hexane-1,6-diamine (HDA), undecane-1,11-diamine, 1-amino-3,5,5-trimethyl-5-aminomethylcyclohexane (IPDA), bis(p-aminocyclohexyl)methane (PACM), 1,5-diamino-2-methylpentane, 2,5-diamino-2,5-dimethylhexane, 1,4-diaminocyclohexane, 2,4- hexahydrotoluylenediamine, 2,6-hexahydrotoluylenediamine (H6TDA), 1,3-bis(aminomethyl)benzene (m-XDA), 1,4-bis(aminomethyl)benzene (p-XDA), isomers of bis(aminomethyl)cyclohexane, isomers of bis(aminomethyl)norbornane (NBDA), neopentanediamine, 2,4,4-trimethylhexamethylenediamine and 2,2,4-trimethylhexamethylenediamine.

Most particularly preferred amines according to the invention are selected from the group consisting of pentane-1,5-diamine (PDA), hexane-1,6-diamine (HDA), 1-amino-3,5,5-trimethyl-5-aminomethylcyclohexane (IPDA), 1,3-bis(aminomethyl)benzene (m-XDA), isomers of bis(aminomethyl)cyclohexan (H6-XDA), isomers of bis(aminomethyl)norbornane (NBDA), 2,4,4-trimethylhexamethylenediamine and 2,2,4-trimethylhexamethylenediamine. Aliphatic amines selected from the group consisting of pentane-1,5-diamine and hexane-1,6-diamine are the most suitable. These exhibit high miscibility with water, which is the compound with a lower boiling point preferably present.

Substances with higher boiling points compared to the amine that can occur are for example dimers or oligomers of the amine, which can form while cleaving off ammonia from the amine. This is a side reaction that frequently occurs in hydrogenation processes for example. In particular in the preferred case, in which PDA is present in the composition as an aliphatic amine produced by biotechnological means, the composition may also contain carbohydrates which have not been completely converted in the fermentation process and salts that originate from the fermentation broth and are now still dissolved in the composition. If the composition originates for example from the chemical recycling of PA66 by hydrolysis, it may for example also contain oligomeric fragments of PA66 as a compound having a higher boiling point. Of course mixtures of different substances with a higher boiling point than the amine may also always occur. Unless indicated otherwise, statements of contents specify the mass of the totality of the substances having a higher boiling point than the amine, based on the mass of the composition concerned.

### Step b)

According to the first embodiment in accordance with the invention, the distillation of the composition provided in step a) may for example be performed in a distillation apparatus (1) as sketched in Fig. 1. According to the second embodiment in accordance with the invention, distillation may for example be performed in a distillation apparatus (1) as sketched in Fig. 2. The most important apparatuses in the distillation arrangements are
- a first distillation column (2) equipped with at least one device for delivering heat,
- a second distillation column (4) which optionally likewise has a device for delivering heat
- and an evaporator-condenser (3), which acts as a condenser for the vapours from the first distillation column and as an evaporator for the feed of the second distillation column (first embodiment in accordance with the invention) or as a condenser for the vapours from the first distillation column and as an evaporator for the bottom of the second distillation column (second embodiment in accordance with the invention).

### The other reference characters in the drawings are

- F: Feed
- S1: Bottom product from the first distillation column
- S2: Bottom product from the second distillation column to the evaporator-condenser
- B1: Stream of vapours from the first distillation column
- B2: Stream of vapours from the second distillation column
- L: Condensed low boilers from the second distillation column
- K: Condensate from the condensing space of the evaporator-condenser
- V: Stream of steam from the evaporation space of the evaporator-condenser
- R: Residue
- N1: Non-condensible stream from the evaporator-condenser
- N2: Non-condensible stream from the second distillation column
- P: Product stream (purified amine)

In the distillation columns, a falling liquid phase and a rising steam phase are brought together in counterflow. In the process, heat and mass transfer occurs, which causes the substances to separate. The columns preferably contain separation supporting structures to enlarge the mass transfer area. These structures may for example be different kinds of mass transfer trays (bubble cap trays, sieve trays, valve trays, etc.), packings with a random arrangement of packing material or structured packings, the latter usually involving a low pressure loss and therefore being preferred. The separation performance of a distillation column is characterised by the number of its theoretical trays, i.e. the number of mass transfer trays on which there is an equilibrium between the liquid running down and the steam rising. Since no equilibrium is in reality achieved on a tray, and there are no real trays in packed columns, the number of trays physically present is usually different from the number of theoretical trays, but the concept of theoretical trays can still be transferred to all separation supporting structures, so that in the present case it is convenient to speak in general terms of theoretical stages (or synonymously of theoretical trays), where a theoretical stage causes enrichment in accordance with the thermodynamic equilibrium.

The composition is processed in an apparatus according to Fig. 1 or a similar apparatus, preferably continuously, as described below. The precise operating conditions to be set for the most efficient operation of the distillation apparatus are of course dependent on the nature of the composition to be processed.

### First distillation column

The composition including the amine and at least one compound having a lower boiling point than the amine and at least one compound having a higher boiling point than the amine is usually conveyed by means of a pump and preferably introduced as a liquid feed stream (F) into the first distillation column (2), where it is distributed by a manifold across the cross-section of the column. The optimum position for its introduction, i.e. at the level of which theoretical separation stage this is done depends on the precise composition of the feed stream and other distillation conditions and can be determined by the skilled person according to known rules, if necessary also by means of preliminary experimentation and model calculations. The feed stream can optionally be heated before its entry into the column. In this way, profitable use can be made for example of exhaust heat from a different process or process step.

At the bottom of the column there is at least one device for delivering heat, with which part of the bottoms is evaporated. This may be a tube bundle in the interior of the column bottom, which is for example heated by means of water vapour. It is preferably an external circulation evaporator, i.e for example a tube bundle heat exchanger, into which a bottom stream is either drawn in automatically (natural circulation evaporator) or is conveyed in by means of a pump (forced circulation evaporator) and which is heated for example by means of condensing water vapour. The device for delivering heat causes part of the bottoms to be evaporated and to rise in the column as steam, in the course of which it comes into contact with the liquid running down. In this way, the feed, i.e. the composition, is separated into at least two streams, a stream of vapours (B1) which is enriched with the at least one compound having a lower boiling point than the amine, and a bottom product (S1) which is depleted in this compound. These two streams are then introduced at different positions of the evaporator-condenser (3) in the first embodiment according to the invention. In the second embodiment of the invention, the bottom product (S1) is introduced not into the evaporator-condenser (3), but into the second distillation column (4). Instead, a stream or partial stream from the bottom of the second column is introduced into the evaporator-condenser (3).

The first distillation column (2) is operated at a head pressure in the range from 1 to 23 bar(a), preferably in the range from 2 to 11 bar(a), particularly preferably in the range from 3 to 8 bar(a). It is preferable for this to result in each case in a bottom temperature of 105° C to 240° C, particularly preferably between 120° C and 190° C and most particularly preferably between 135° C and 175° C.

### Evaporator-condenser

In the first embodiment of the invention, the stream of vapours (B1) removed from the head of the first distillation column (2) is introduced into the condensing space of the evaporator-condenser, while the bottom product (S1) removed from the bottom of the first distillation column (2) is fed to the evaporation space of the evaporator-condenser (3). Prior to or upon its entry into the evaporation space, the pressure of the bottom product is reduced to a pressure between the head pressure of the first distillation column and the head pressure of the second distillation column.

In the second embodiment of the invention, the stream of vapours (B1) removed from the head of the first distillation column (2) is introduced into the condensing space of the evaporator-condenser, while a stream or partial stream (S2) removed from the bottom of the second distillation column (4) is fed to the evaporation space of the evaporator-condenser (3).

Irrespective of the embodiment, the pressure in the evaporation space is lower than the pressure in the condensing space, which results as a rule from the difference between the head pressures of the first and second distillation columns. Because of the pressure losses occurring in the vapour feed line from the first column into the condensing space of the evaporator-condenser on the one hand and in the feed line from the evaporation space into the second column on the other, the pressure difference between the condensing space and the evaporation space of the evaporator-condenser is usually somewhat lower than the difference between the head pressures of the two distillation columns. It is preferably ≥ 0.3 bar and ≤ 22.0 bar, particularly preferably ≥ 1.0 bar and ≤ 10.0 bar and most preferably ≥ 2.0 bar and ≤ 8.0 bar.

It is preferable to select the pressure in the condensing space so much lower that a logarithmic mean temperature difference of ≥ 5 K and ≤ 180 K, preferably ≥ 7 K and ≤ 150 K, particularly preferably ≥ 11 K and ≤ 130 K, very particularly preferably ≥ 18 K and ≤ 110 K and most preferably ≥ 25 K and ≤ 100 K is established between the condensing space and the evaporation space.

The stream of vapours (B1) is largely, meaning more than 60%, preferably more than 80% and particularly preferably more than 90%, condensed in the evaporator-condenser and the condensation heat released in the process is passed to the evaporation space, where it leads to the partial evaporation of the liquid present therein. A stream of non-condensed portions (N) is discharged from the condensing space of the evaporator-condenser. That is preferably fed, together with the stream of vapours from the second distillation column, to the condenser associated with that column so that further portions can be condensed out of it there. This for example prevents non-condensible inert substances from accumulating in the evaporator-condenser and impairing its performance.

The condensate from the condensing space of the evaporator-condenser can be fed at least partially, preferably completely, as back feed into the first and/or second distillation column. It is preferably divided into at least two partial streams, one of which is returned to the first distillation column as back feed and is preferably introduced there above the first separation stage, and a further one of which is fed as back feed to the second distillation column. A possible further partial stream can be recovered from the distillation apparatus and processed otherwise, e.g. by distillation, and/or disposed of. The back feed to the second distillation column can, but does not have to, be introduced above the first separation stage. Introducing it on a lower separation stage increases the purity of the distillate but at the expense of higher energy consumption.

According to the first embodiment of the invention, the bottom product from the first distillation column is partially evaporated in the evaporation space of the evaporator-condenser, which results in at least two streams emerging from the evaporation space of the evaporator-condenser. These are a vapour stream (V), which is fed as feed to the second distillation column, and a residual stream (R), which substantially contains the amine and the at least one compound having a higher boiling point than the amine. This stream can be recovered either directly at the evaporator-condenser or in a downstream gas-liquid separator and is first removed from the distillation apparatus. Possible methods for the further treatment and optional return of this stream to the distillation apparatus will be described later.

In the second embodiment of the invention, the evaporator-condenser serves as an evaporator for the bottoms of the second column, i.e. a liquid stream taken from the bottom of the column or a partial stream (S2) of a liquid stream taken from the bottom is at least partially condensed in the evaporation space of the evaporator-condenser and then at least partially returned to the second distillation column. Part of the steam phase generated can be discharged as product stream (P) at this point. Non-evaporated amounts from the evaporation space of the evaporator-condenser can likewise be returned to the column and/or discharged as a residual stream (R). The residual stream (R) substantially contains the amine and the at least one compound having a higher boiling point than the amine. Depending on the composition and the operating conditions, it may happen that the liquid stream taken from the bottom of the column contains small amounts of solids. The content by weight of solids in this stream is usually ≤ 5 wt.-%, preferably ≤ 2 wt.-% and particularly preferably ≤ 1 wt.-%. Under the operating conditions, the stream is preferably free of solids.

Irrespective of the embodiment concerned, it is also possible to withdraw a liquid stream, optionally including small amounts, i.e. less than 5 wt.-%, preferably less than 2 wt.-% and particularly preferably less than 1 wt.-%, from the evaporation space of the evaporator-condenser or lines or apparatuses connected to it, and to return them to the apparatus. The liquid load on the apparatus can be actively controlled in this way.

### Second distillation column

The vapour stream (V) from the evaporation space of the evaporator-condenser is fed to the second distillation column (4), where the fractionation of the composition is completed. At the head of the second distillation column, a stream of vapours (B2) is recovered which contains the at least one compound having a lower boiling point than the amine. This is at least partially condensed in a condenser in order to obtain a liquid stream (L) enriched in lower boiling compound compared to the amine. This can then be removed from the distillation apparatus. It is preferable for this to be fed at least partially as back feed stream to the second and/or first distillation column, particularly preferably to the second distillation column. If it is returned completely as back feed, the column requires a lateral removal point where a liquid stream of low boilers is recovered. Non-condensed portions of the vapours are preferably released into the atmosphere after waste-gas treatment, such as gas scrubbing, thermal treatment or activated-carbon filtration.

The amine to be obtained can be recovered in the first embodiment of the invention below the feed either as a side stream, preferably as a liquid side stream, or as a bottom product (product stream (P)). As a rule, it is preferable to recover the amine as a bottom product. Recovery as a side stream between the feed inlet and the bottom of the column makes sense for example whenever a high boiler has a boiling point close to that of the amine and therefore too large an amount of this high boiler has been introduced into the column with the gaseous feed.

In the second embodiment of the invention, in which the evaporator-condenser is operated as an evaporator for the bottoms of the second distillation column, the amine to be obtained can be recovered as a partial stream from the steam generated in the evaporation space of the evaporator-condenser or as a side stream below the feed from the second distillation column. If the purified amine is recovered as a side stream from the distillation column, it can be recovered either as a liquid stream or as a gaseous stream, preferably as a vaporous stream. The product stream (P) can be put to further use as it stands or if necessary subjected to a further vacuum distillation process in order to separate any compounds with higher boiling points that might have been entrained and to purify the amine further in this way.

Irrespective of the embodiment concerned, the second distillation column (4) is operated at a head pressure in the range from 0.05 to 1.4 bar(a), preferably in the range from 0.06 to 1.1 bar(a), particularly preferably in the range from 0.07 to 0.7 bar(a) and most preferably in the range from 0.08 to 0.4 bar(a). It is preferable for this to result in each case in a bottom temperature of 50° C to 180° C, particularly preferably between 70° C and 150° C and most particularly preferably between 80° C and 175° C.

At the bottom of the second column there is optionally a further device for delivering heat, with which part of the bottoms can be evaporated. This may be a tube bundle in the interior of the column bottom or an external circulation evaporator. Heating is carried out for example by means of condensing water vapour. In standard operation, such additional heating is not vitally necessary, but it is very useful for commissioning processes for example, in order to compensate for process fluctuations or to achieve a particularly low water content in the product stream.

The at least one compound which has a higher boiling point than the amine and is contained in the composition provided in step a) is enriched during distillation and ultimately leaves the distillation system with the residual stream (R).

### Further embodiments

In one further embodiment of the invention, the stream of vapours from the second distillation column is used to heat other product streams and/or to evaporate solvents.

In another preferred embodiment of the invention, residual stream (R) is further concentrated, with the recovery of amine. It is preferred for this to be done in a thermal process, in which the amine contained is at least partially evaporated. It is expeditious in this context and thus particularly preferable to perform the evaporation at a low pressure, for example in the range from 0.001 to 0.500 bar(a), preferably in the range from 0.001 to 0.050 bar(a) and particularly preferably in the range from 0.002 to 0.03 bar(a). In this way, the temperature needed to evaporate the amine can be lowered for many amines preferably to less than 120° C, particularly preferably less than 100° C and most particularly preferably less than 70° C, so that the concentration process is mild, and problems with deposits of solids because of thermal decomposition or polymerisation for example of the compound having a higher boiling point than the amine are kept to a minimum or prevented altogether. The evaporated amine from this residue concentration is separated from the residue and can preferably be returned to the distillation apparatus after condensation. The stream is preferably integrated before or in the first distillation column or before or in the evaporation space of the evaporator-condenser.

With the combination of distillation steps according to the invention, the product stream obtainable with the method is different. Hence, a further subject-matter of the invention is a composition including the amine, obtainable or produced using the method of the invention according to the first and/or the second embodiment and the preferred embodiments in each case. Thanks to the particularly advantageous composition of the product stream obtainable according to the method of the invention and containing the amine, it is particularly well suited to the production of isocyanates by phosgenation.

Another subject-matter of the invention is a method for the production of an isocyanate, comprising converting the composition of the invention with phosgene. The phosgenation of the amine may for example be performed in the gaseous phase. The skilled person is sufficiently familiar with methods for the gas phase phosgenation of amines from the state of the art. Phosgenation is performed at temperatures ranging from 200 to 600° C with an excess of phosgene, optionally in the presence of an inert gas or vapours of an inert solvent such as chlorobenzene or o-dichlorobenzene.

The phosgene used in phosgenation is preferably employed in an excess, based on the amino groups. In general an amount of phosgene corresponding to 150 to 350% of the theory concerning the phosgenation reaction taking place is sufficient. The phosgene stream is preferably heated to a temperature within the range from 200 to 600° C before the reaction.

After conversion, which is performed in a preferably cylindrical reaction space, the isocyanate formed is removed from the reaction mixture, preferably by selective condensation in an inert solvent such as chlorobenzene or dichlorobenzene, and is then processed into pure isocyanate in a multiple distillation process.

In a further embodiment, the phosgenation of the amine takes place in the liquid phase. The reaction can then be performed in various ways. Either the amine is reacted directly with an excess of phosgene in an inert liquid medium, preferably in a two-stage process known as cold-hot phosgenation (base phosgenation) or it is first transformed into the corresponding salt by conversion with hydrogen chloride gas or carbon dioxide in an inert liquid medium and then converted with an excess of phosgene similar to the hot phosgenation step in base phosgenation (hydrochloride or carbaminate phosgenation). A suitable liquid medium for all phosgenations is in particular chlorobenzene and/or dichlorobenzene. The skilled person is also familiar with these processes for phosgenation in the liquid phase.

Both in base phosgenation and in amine-hydrochloride or carbaminate phosgenation, the remaining phosgene and hydrogen chloride gas is preferably blown off with an inert gas, preferably nitrogen, after the reaction is completed. If needed, filtration may be performed in order to remove any solids that might be present, such as unreacted amine-hydrochlorides.

Irrespective of the choice of phosgenation process, the isocyanate formed is then processed by multiple distillation to the pure isocyanate.

If the amine is an amine with two or three NH₂ groups per molecule, the amine obtained by the method of the invention can as an alternative to the reaction with phosgene described above also be reacted with at least one epoxy resin or used as a raw material in the production of polyamide.

The present invention will be explained in more detail with reference to the following examples, though without being limited thereto.

### Examples

The invention will now be explained with reference to examples. All the examples are process simulations which were performed using Aspen Plus^{®}. As the composition from which the amine is to be obtained, the following compositions were taken as the basis for the simulations:
Composition A:
10.2 wt.-% pentamethylenediamine, 88.1 wt.-% water as the compound having a lower boiling point than the amine, 0.6 wt.-% compounds having higher boiling points than the amine and 1.1% further secondary components having lower boiling points than the amine, including inert gases.

### Comparative example 1:

A distillation process for composition A was modelled on the teaching of EP1602640A1. While it was possible to reduce the energy consumption considerably by using the heat of the steam from the column operated with a higher head pressure compared to distillation without this energy integration, the product nevertheless contained approx. 3 wt.-% water, approx. 92 wt.-% PDA and approx. 5 wt.-% impurities with higher boiling points and was not suitable for further use in phosgenation, especially gas phase phosgenation to pentamethylene diisocyanate. The water content at the bottom of the last column was too high for such an application, which would cause corrosion problems in the phosgenation apparatus. While it would be conceivable to reduce the water content by making greater use of heating steam, this would nevertheless result in an increase in the bottom temperature and would have a negative effect on the thermal integration of the two columns. Furthermore, the proportion of compounds having a higher boiling point in the purified amine was beyond all specifications for amines for the production of isocyanate, because there was no separation of residues, and would cause problems in the evaporation of the amine precisely in gas phase phosgenation.

### Comparative example 2:

For a comparison with a classic distillation sequence for aliphatic diamines, with which the skilled person is familiar, the removal of water from composition A in a single distillation column was also simulated. A column with 50 theoretical stages and a partial condenser with a back feed divider was assumed. Distillation was performed at 1 bar(a) head pressure. 4 t/h of the composition were preheated to 95° C and then introduced into the 20th stage of the column. The return ratio (back feed : distillate) was set at 0.12. At the bottom of the column, the heating power was adjusted such that the amine was produced with a water content of 500 ppm. Under these conditions, a heating power of 2528 KW was required for this purpose. The specific energy consumption was thus 5.82 kWh/kg based on the amine stream with water removed, but for any further use, it needed further purification in a phosgenation reaction.

### Example 1a: Performed in an apparatus according to Fig. 1

Distillation of composition A was modelled in accordance with the teaching of the present invention in a distillation apparatus comprising a first distillation column, an evaporator-condenser and a second distillation column.

A feed stream of 4 t/h of composition A was heated in a preheating step from 40° C to 95° C and fed into the 20th stage of the first distillation column (50 theoretical stages, circulation evaporators, no condenser). The head pressure in the column was fixed at 3.0 bar(a) and a heating power of 1395 KW was delivered to the bottom of the column. At the head of the first distillation column, a gaseous stream consisting substantially of water was recovered at a temperature of 133° C and fed into the condensing space of the evaporator-condenser. The bottom product of the first distillation column was 2453 kg/h and contained 16.7 wt.-% pentamethylenediamine, together with 1.0 wt.-% high boilers, traces of low boilers and 82.3 wt.-% water. When it entered the evaporator-condenser, its pressure was lowered to 330 mbar(a), as a result of which part of the stream already evaporated and an inlet temperature of 72° C was established. The stream was then, and in the evaporation space of the evaporator-condenser, heated at an unchanged pressure and evaporated almost completely. The gas stream which formed had a temperature of 90° C at the exit from the evaporation space. In the condensing space, the stream of vapours from the first column was cooled to 122° C, largely condensing in the process, and thus provided the heat for the evaporation in the evaporation space. A quarter of the condensate obtained was introduced at the head of the first distillation column as a back feed stream. The remaining part was discharged in this example (in Example 1a it was used as an additional back feed for the second distillation column). A residual stream of 41.3 kg/h including approx. 30% pentamethylenediamine, approx. 10% water and approx. 60 wt.-% high boilers was discharged in liquid form from the evaporation space of the evaporator-condenser. The steam phase obtained in the evaporation consisted substantially of water (83.5 wt.-%) and pentamethylenediamine (16.4 wt.-%) and was fed as steam to the 30th stage in the second distillation column (50 theoretical stages, circulation evaporators, total condenser with back feed divider), which was operated at a head pressure of 220 mbar. The condensation of the vapours was performed completely at 62° C and a return ratio (back feed : distillate) of 0.14 was chosen. A heating power of 113 KW was delivered to the bottom of the second distillation column. At the bottom of the second column, 396 kg/h of the purified amine with a purity of more than 99.97 wt.-% was obtained, which is suitable for the production of isocyanates in (gas phase) phosgenation.

In total, the heating power required by the two columns was 1.51 MW and hence even less than only the evaporation enthalpy of the water contained in the first composition, which in a standard amine distillation sequence would have to be applied just in the dewatering column itself without taking into account any back feed that might be necessary. Thanks to the thermal integration, a power of 1.20 MW from the waste heat from the steam condensation could be utilised in the evaporator-condenser. The specific energy consumption was thus 3.81 kWh/kg based on the pure amine.

The logarithmic mean temperature difference of the evaporator-condenser calculated theoretically from the input and outlet temperatures was 46 K.

### Example 1b:

The simulation of Example 1a was modified, so that the partial stream of the condensate discharged in Example 1a was now introduced at the head of the second column as additional back feed. Because of the inert gases contained in this stream, condensation was no longer complete even at 52° C, but instead a small stream of non-condensible gases was discharged. The return ratio of the second distillation column (back feed : distillate) was lowered to 0.08 and the following further operating data resulted:
The specific energy consumption was 3.82 KWh/kg based on the pure amine and a logarithmic mean temperature difference of 46 K at the evaporator-condenser was calculated from the input and outlet temperatures.

### Example 1c:

The simulation from Example 1a was modified such that the liquid residual stream discharged from the evaporation space of the evaporator-condenser was fed to a further flash evaporator. The mass flow of this stream was 171 kg/h and it still contained 65.3 wt.-% pentamethylenediamine. In the newly added flash evaporator with a heating power of 34 KW, evaporable portions were evaporated from the stream at a pressure of 10 mbar and a temperature of approx. 59° C and the remaining liquid residue was removed from the distillation system. This mass flow of 33.3 kg/h still contained approx. 27 wt.-% pentamethylenediamine and 0.5 wt.-% water. The evaporated portions were condensed completely and fed back by means of a pump to the lowest stage of the first distillation column. At the evaporator-condenser, the gas exit temperature from the evaporation space was 82° C and at the bottom of the second distillation column 400 kg/h of the purified amine (99.99 wt.-%) were recovered.

The specific energy consumption was 3.91 KWh/kg based on the pure amine and a logarithmic mean temperature difference of 50 K at the evaporator-condenser was calculated from the input and outlet temperatures.

### Example 2: Performed in an apparatus according to Fig. 2

Distillation of composition A was modelled in accordance with the teaching of the present invention in a distillation apparatus comprising a first distillation column, an evaporator-condenser and a second distillation column.

A feed stream of 4 t/h of composition A was heated in a preheating step from 40° C to 95° C and fed into the 20th stage of the first distillation column (50 theoretical stages, circulation evaporators, no condenser). The head pressure in the column was fixed at 7.6 bar(a) and a heating power of 1553 KW was delivered to the bottom of the column. At the head of the first distillation column, a gaseous stream consisting substantially of water was recovered at a temperature of 168° C and fed into the condensing space of the evaporator-condenser. There it was cooled to an exit temperature of 156° C, in the course of which it largely condensed and released a condensation heat of 1189 KW to the evaporation space. A quarter of the condensate obtained was introduced at the head of the first distillation column as a back feed stream. The remaining part was discharged in this example. The bottom product of the first distillation column was 2404 kg/h and contained 17.0 wt.-% pentamethylenediamine, together with approx. 1.0 wt.-% high boilers, traces of low boilers and 81.9 wt.-% water. It was delivered as feed to the 30th stage of the second distillation column (50 theoretical stages, total condenser with back feed divider), which was operated at a head pressure of 220 mbar. At stream of liquid exiting the bottom of the column had a temperature of 133° C and was fed into the evaporation space of the evaporator-condenser. There it was heated to an exit temperature of 156° C by utilising the condensation heat and largely evaporated in the process. The portion which was not evaporated was discharged from the system as a residual stream. The residual stream had a mass flow of 38.1 kg/h and still contained approx. 37.5% pentamethylenediamine in addition to high boilers. The evaporated portion was again divided into two partial streams, one of which was discharged as a product stream. That had a mass flow of 395 kg/h and contained the purified amine in a purity of 99.92%. The other partial stream was fed back in gaseous form to the bottom of the second distillation column in order to heat it. In addition to that, no further heating power was used for the second distillation column. The vapours exiting at the head of this column were condensed completely at 62° C and partly introduced as back feed at the head of the column according to the chosen return ratio (back feed : distillate) of 0.1 and partly discharged from the process.

In this example, the entire external heating power required for the process was thus delivered to the bottom of the first distillation column. As previously described, this was 1.55 MW and hence even less than only the evaporation enthalpy of the water contained in the first composition, which in a standard amine distillation sequence would have to be applied just in the dewatering column itself without taking into account any back feed that might be necessary. Thanks to the thermal integration, a power of 1.19 MW from the waste heat from the steam condensation could be utilised in the evaporator-condenser. The specific energy consumption was thus 3.93 kWh/kg based on the pure amine.

The logarithmic mean temperature difference of the evaporator-condenser calculated theoretically from the input and outlet temperatures was 17 K.

### Discussion of the examples:

The examples show that distillation according to the first embodiment of the invention, just like distillation according to the second embodiment of the invention, enables a considerable energy saving compared to a classic distillation sequence thanks to the thermal integration in the evaporator-condenser. In addition, Examples 1 and 2 make it clear that the first embodiment of the invention enables a milder distillation of the amine. The head pressure in the first distillation column in Examples 1a-c could be set at a considerably lower level for example, and still a greater logarithmic mean temperature difference was achieved at the evaporator-condenser than in Example 2. Thanks to the lower head pressure, the temperature at the bottom of the column is also lower, so that the thermal load on the amine is reduced. In the case of insensitive amines on the other hand, the first embodiment allows a higher head pressure in the second distillation column, so that the condensation heat of its vapours could also be reused in order, for example, to generate water vapour for other processes. Compared to the distillation process according to EP1602640A1, the embodiments of the invention display an advantage regarding the purity of the amine obtained. Example 1c shows that with an additional residue concentration step, the operating parameters can be adapted in such a way that the stream of high boilers is produced at a particularly low temperature. This can be advantageously exploited especially in the case of thermally unstable residues.

## Claims

1. Method for producing an amine comprising the steps of:
a) providing a composition comprising the amine, at least one compound having a lower boiling point than the amine and at least one other compound having a higher boiling point than the amine,
b) distilling the composition in a thermally integrated distillation apparatus comprising a first distillation column, an evaporator-condenser with an evaporation space and a condensing space and a second distillation column, wherein
• the composition of step a) is introduced as feed into the first distillation column operating at a head pressure in the range from 1 to 23 bar(a),
• a bottoms liquid depleted in the lower boiling compound from the first distillation column is introduced into the evaporation space of the evaporator-condenser, is partially evaporated and is then separated into a gaseous feed, which is introduced into the second distillation column, and a residual stream residual stream,
• the second distillation column is operating at a head pressure between 0.05 to 3.8 bar(a) and at least 0.5 bar which is at least 0.5 lower than the head pressure in the first distillation column,
• a first stream of vapours from the first distillation column is introduced into the condensing space of the evaporator-condenser, is at least partially condensed there so that a condensate forms and the condensation heat is used to evaporate the bottoms liquid of the first column in the evaporation space of the evaporator-condenser partially, and
• a product stream including the amine is removed as a side stream below the feed or as a bottoms product from the second column.

2. Method for producing an amine comprising the steps of:
a) providing a composition comprising the amine, at least one compound having a lower boiling point than the amine and at least one other compound having a higher boiling point than the amine,
b) distilling the composition in a thermally integrated distillation apparatus comprising a first distillation column, an evaporator-condenser with an evaporation space and a condensing space and a second distillation column, wherein
• the composition of step a) is introduced as feed into the first distillation column operating at a head pressure in the range from 1 to 23 bar(a),
• the bottoms liquid from the first distillation column depleted in the lower boiling compound is introduced as feed into the second distillation column which is operating at a head pressure in the range from 0.05 and 1.5 bar(a) which is at least 0.5 bar below the head pressure in the first distillation column,
• a liquid stream is removed from the bottoms of the second distillation column and is introduced completely or partially into the evaporation space of the evaporator-condenser, is partially evaporated therein thus generating steam which is at least partially fed back into the column,
• a liquid residual stream which may optionally comprise solids is removed and discharged from the evaporation space of the evaporator-condenser, from the bottom of the column or as part of the bottoms liquid removed there,
• a steam stream from the first distillation column is introduced into the condensing space of the evaporator-condenser, and is at least partially condensed therein, thus forming a condensate, and the condensation heat is used to partially evaporate the liquid stream removed from the bottoms of the column, and
• a product stream including the amine is removed either as a partial stream from the steam produced in the evaporation space of the evaporator-condenser or is removed below the feed as a side stream from the second distillation column.

3. The method according to either of claims 1 or 2, **characterised in that** the composition provided in method step a) comprises ≥ 40 wt.-% and ≤ 99 wt.-%, preferably ≥ 60 wt.-% and ≤ 98 wt.-% and particularly preferably ≥ 70 wt.-% and ≤ 95 wt.-% of the at least one compound having a lower boiling point than the amine.

4. The method according to any one of claims 1 to 3, **characterised in that** the composition comprises ≥ 1 wt.-% and ≤ 50 wt.-%, particularly preferably ≥ 2% wt.-% and ≤ 40 wt.-% and most particularly preferably ≥ 4 wt.-% and ≤ 25 wt.-% of the aliphatic amine.

5. The method according to any one of claims 1 or 4, **characterised in that** the composition comprises ≥ 0.01 wt.-% and ≤ 10 wt.-%, preferably ≥ 0.1 wt.-% and ≤ 5 wt.-% and most particularly preferably ≥ 0.3 wt.-% and ≤ 3 wt.-% of the at least one compound having a higher boiling point than the amine.

6. The method according to any one of claims 1 to 5, **characterised in that** the at least one compound having a lower boiling point than the amine is water.

7. The method according to any one of claims 1 to 6, **characterised in that** the composition comprises ≥ 40 wt.-% and ≤ 99 wt.-%, preferably ≥ 60 wt.-% and ≤ 98 wt.-% and particularly preferably ≥ 70 wt.-% and ≤ 95 wt.-% water.

8. The method according to any one of claims 1 to 7, **characterised in that** the amine is selected from the group consisting of diaminotoluene, n-butylamine, iso-butylamine, n-hexylamine, butane-1,4-diamine, pentane-1,5-diamine, hexane-1,6-diamine, undecane-1,11-diamine, 1-amino-3,5,5-trimethyl-5-aminomethylcyclohexane, bis(p-aminocyclohexyl)methane, 1,5-diamino-2-methylpentane, 2,5-diamino-2,5-dimethylhexane, 1,4-diaminocyclohexane, 2,4-hexahydrotoluylenediamine, 2,6-hexahydrotoluylenediamine, 1,3-bis(aminomethyl)benzene, 1,4-bis(aminomethyl)benzene, isomers of bis(aminomethyl)cyclohexane, isomers of bis(aminomethyl)norbornane, neopentanediamine, 2,4,4-trimethylhexamethylenediamine and 2,2,4-trimethylhexamethylenediamine.

9. The method according to any one of claims 1 to 8, **characterised in that** the first distillation column is operated at a head pressure in the range from 2 to 11 bar(a), preferably in the range from 3 to 8 bar(a).

10. The method according to any one of claims 1 to 9, **characterised in that** the condensate from the condensing space of the evaporator-condenser is fed at least partially, preferably completely, as back feed into the second distillation column.

11. The method according to any one of claims 1 to 10, **characterised in that** the second distillation column is operated at a head pressure in the range from 0.05 to 1.4 bar(a), preferably in the range from 0.06 to 1.1 bar(a), particularly preferably in the range from 0.07 to 0.7 bar(a) and most preferably in the range from 0.08 to 0.4 bar(a).

12. The method according to any one of claims 1 to 11, **characterised in that** the residual stream is further concentrated by at least partial evaporation of the amine contained therein and that said concentration process is preferably performed at a pressure in the range from 0.001 to 0.500 bar(a), particularly preferably in the range from 0.001 to 0.050 bar(a) and most particularly preferably in the range from 0.002 to 0.03 bar(a).

13. The method according to claim 12, **characterised in that** the evaporated amine is separated from the residue, then condensed and fed back into the distillation apparatus.

14. Composition comprising the amine, obtained or obtainable according to one of the methods according to any one of claims 1 to 13.

15. Method for producing an isocyanate, comprising reacting the composition according to claim 14 with phosgene.
